# EUROPÄISCHE PATENTSCHRIFT

(11) **EP 0 941 318 B1**
(45) Veröffentlichungstag und Bekanntmachung des Hinweises auf die Patenterteilung: **17.01.2001**
(21) Anmeldenummer: 97949949.8
(22) Anmeldetag: 13.11.1997
(51) Int. Cl.: C12N 15/11, C12N 15/89, A61K 48/00

(54) **HANTELFÖRMIGE EXPRESSIONSKONSTRUKTE FÜR DIE GENTHERAPIE**
DUMBBELL EXPRESSION CONSTRUCTS FOR GENE THERAPY
CONSTRUCTIONS DE STRUCTURES EN HALTERE POUR EXPRESSION EN THERAPIE GENIQUE

(30) Priorität: 13.11.1996 DE 19648625
(43) Veröffentlichungstag der Anmeldung: 15.09.1999
(73) Patentinhaber: Soft Gene GmbH, 14195 Berlin (DE)
(72) Erfinder: WITTIG, Burghardt, D-14197 Berlin (DE); JUNGHANS, Claas, D-10587 Berlin (DE)
(74) Vertreter: Omsels, Hermann-Josef
(86) Internationale Anmeldenummer: DE9702704
(87) Internationale Veröffentlichungsnummer: WO9821322

(56) Entgegenhaltungen:
- EP-A- 0 686 697
- WO-A-96/32473

## Beschreibung

Die Erfindung betrifft ein Design-Prinzip für ein minimales Expressionskonstrukt, welches neben Promotor- und Terminationssequenzen, die zur Steuerung der Expression nowendig sind, keine andere genetische Information enthält als die zu exprimierende. Derartige minimale Expressionskonstrukte sollen für molekularmedizinische Anwendungen eingesetzt werden, insbesondere die genetische Vakzination, Onkotherapie und -Prophylaxe.

Das Design-Prinzip soll zur Konstruktion von Expressionskonstrukten für die Expression von MHC-I oder MHC-II präsentierbaren Peptiden, Cytokinen oder Komponenten der Regulation des Zellzyklus, oder zur Synthese regulativer RNA-Moleküle wie antisense-RNA, Ribozymen oder mRNA-editierende-RNA eingesetzt werden. Wesentlicher Bestandteil der Erfindung ist es zudem, daß das Konstruktionsprinzip die kovalente Verknüpfung des Expressionskonstrukts erlaubt, so zum Beispiel mit Peptiden, Proteinen, Kohlenhydrat- oder Glycopeptidliganden, sowie Partikeln, die zum ballistischen Transfer zum Einbringen der Konstrukte in die Zellen, insbesondere der Dermis, des Muskelgewebes, des Pankreas und der Leber benutzt werden können.

Die Erfindung soll insbesondere für zwei eng miteinander verwandte Gebiete benutzt werden: somatische Gentherapie und genetische Vakzinierung. In der Immuno-Gentherapie onkologischer Erkrankungen berühren sich diese Gebiete. Während bei der "klassischen" Gentherapie fehlende oder fehlerhafte Gene ersetzt werden sollen, soll bei der immunotherapeutischen Gentherapie das Immunsystem des Patienten gegen tumorspezifische Antigene aktiviert werden. Beim malignem Melanom und einigen anderen Tumoren konnte eine Reihe von tumorspezifischen Antigenen identifiziert werden, die durch cytotoxische T-Lymphocyten erkannt werden (Van den Eynde B. and Brichard V. G., Current Opinion in Immunology (1995) 7: 674-681). In den meisten Fällen handelt es sich um Fragmente mutierter Proteine, die entweder für die Tumorentwicklung und Promotion relevant sind (Stüber et al., Eur. J. Immunol. (1994) 24: 765-768), oder um Fragmente von Proteinen aus dem veränderten Stoffwechsel der Tumorzelle. Im Fall des Melanoms entstammen die präsentierten Peptide häufig Proteinen aus der Melanocyten-spezifischen Differenzierung. Für Ansätze, die sich der Aktivierung des Immunsystems gegen solche tumorspezifischen Antigene bedienen, sind Methoden wichtig, die genannten antigenen Epitope auch in nicht-Tumorzellen wie antigenpräsentierenden Zellen (Makrophagen, dendritische Zellen) zu überexprimieren. Alternativ sind auch Gene, die die Expression Peptid-präsentierender Proteine steuern, wie CIITA oder ICSBP, von großer Bedeutung.

Laborexperimente und klinische Studien, in denen solche Peptide für die Induktion oder Verstärkung einer tumorspezifischen cytotoxischen Antwort eingesetzt wurden, konzentrieren sich auf konventionelle Vakzinationsprotokolle, bei denen die jeweiligen Peptide eingesetzt wurden (Strominger J., Nature Medicine, (1995) 1: 1.179 - 1.183). Alternativ wurden Antigen-präsentierende Zellen, wie dendritische Zellen, mit hohen Konzentrationen solcher Peptide inkubiert. Dadurch werden die ursprünglich durch den MHC-Komplex präsentierten Peptide gegen die tumorspezifischen Peptide ausgetauscht (Grabbe et al., Immunology Today (1995) 16:117-121).

Der Begriff der genetischen Vakzinierung (Immunisierung) beschreibt die Nutzung eines experimentellen Befundes, der zunächst als Artefakt wissenschaftlich umstritten war, seit kurzer Zeit aber für eine Vielzahl von biomedizinischen Fragestellungen bestätigt werden konnte (Piatak et al., Science 259 (1993): 1745 - 1749). Injiziert man ein Expressionsplasmid für Säugerzellen in Haut- oder Muskelschichten, so kommt es, wenn auch in sehr geringer Effizienz, in den Zellen dicht an der Injektionsstelle zur Expression des entsprechenden Gens. Handelt es sich bei dem Expressionsprodukt um ein Fremdprotein (xenogenes oder allogenes Protein), kommt es, wahrscheinlich im Laufe einer lokalen Entzündung, zur Aufnahme und Präsentation von Fragmenten des exprimierten Proteins (Oligopeptide) durch Antigen-präsentierende Zellen (APCs). Je nach lokaler Cytokin-Situation und in Abhängigkeit davon, in welchen Zellen das Plasmid exprimiert wird (Präsentation über MHC-I oder MHC-II), kommt es zur Auslösung einer Immunreaktion auf dem T_{H1} oder T_{H2} Weg (Wang et al., Human Gene Therapy 6 (1995): 407 - 418), die letztlich jeweils zur Proliferation cytotoxischer T-Zellen oder zur Produktion löslicher Antikörper führen. Die Transfektion von dendritischen Zellen mit Expressionskonstrukten für antigene Peptide *ex vivo* wird in diesem Zusammenhang hier ebenfalls als genetische Vakzinierung bezeichnet.

Eine solche genetische Vakzinierung vermeidet die vielfältigen Risiken herkömmlicher Immunisierungsverfahren. Es sind zahlreiche Ansätze in der Gentherapie bekannt, durch das Einbringen von Erbinformation in Zellen therapeutische oder prophylaktische Wirkungen zu erzielen. Diese Ansätze sind nicht nur im Tierversuch, sondern auch in zahlreichen klinischen Studien an Patienten demonstriert worden, so beispielsweise das sog. ballistomagnetische Vektorsystem (EP 0 686 697 A2) für die Transfektion mit herkömmlichen, plasmidbasierten Expressionskonstrukten. Das ballistomagnetische Vektorsystem wurde durch die hiesige Anmelderin/Erfinder in drei klinischen Phase-I/II Studien für die Produktion Interleukin-7 (IL-7), Interleukin-12 (IL-12) oder Granulocyten-Makrophagen-Koloniestimulierender Faktor (GM-CSF) überexprimierender Tumorzellen eingesetzt. Im Fall der Expression von IL-12 wurden separate Expressionskonstrukte für die Gene der beiden IL-12 Untereinheiten gleichzeitig ballistomagnetisch transfiziert (Schadendorf et al., Molecular Medicine Today 2 (1996): 144-145).

Mit dem Fortschreiten dieser neuen Disziplin muss allerdings der bisherige Methodenkatalog der Gentherapie kritisch untersucht werden. Ein wesentlicher Aspekt ist dabei die auf den bisher verwendeten DNA-Konstrukten enthaltene Sequenzinformation. Sollen Expressionskonstrukte an großen Patientenzahlen, und möglicherweise mehrmals, angewendet werden, so werden Sicherheitsaspekte besonders in immunologischer Hinsicht stark an Gewicht gewinnen. Die bisher angewendeten Expressionskonstrukte sind Weiterentwicklungen eukaryoter Expressionsplasmide. Diese weisen zwei fundamentale Nachteile auf: Ihre Größe, die einen schnellen Transport in den Zellkern behindert, und die Anwesenheit von für die eigentliche Anwendung nicht benötigten Sequenzen. Bisher verwendete Expressionskonstrukte tragen konstitutiv exprimierte Gene z.B. für Cytostatikaresistenzen, die als Selektionsmarker fungieren, sowie ggf. Sequenzen, die zur episomalen Replikation in der Zielzelle notwendig sind. Die Expression dieser Gene stellt einen unerwünschten Hintergrund der transfizierten genetischen Information dar. Zudem tragen diese Konstrukte neben der eigentlichen Promotor-Gen-Terminationsstruktur, die zur Expression gebracht werden soll, mindestens die zur bakteriellen Replikation notwendigen Sequenzen, da die Plasmide in Bakterien propagiert werden, die ebenfalls zur eigentlichen Anwendung nicht benötigt werden.

Es ist offensichtlich, daß die bisherigen Expressionskonstrukte nicht nur zur Expression des eigentlich gewünschten Gens führen, sondern auch zur Biosynthese xenogener Proteine, selbst wenn deren prokaryontische Promotoren sehr niedrige Aktivität in Säugerzellen aufweisen. Bei längerer oder wiederholter Applikation kann davon ausgegangen werden, daß die erwünschte Immunantwort durch solche kontaminierenden Genprodukte maskiert wird und signifikante immunologische Komplikationen entstehen können.

Eine andere Problematik bei der Anwendung gentherapeutischer Methoden betrifft die Methode, mit der das zu transferierende genetische Material in die Zellen hineingebracht wird. Aus Gründen der Effizienz, immunologischen Sicherheit und breiten Anwendbarkeit über viele Zelltypen ist die Methode des ballistischen Transfers bevorzugt. Ein wesentlicher Vorteil des ballistischen Transfers gegenüber den alternativen Transfektionsmethoden ist, daß die Methode relativ leicht zwischen verschiedenen Zell- oder Gewebetypen übertragbar ist. Die heute vielfach angewendeten Methoden zur Transfektion von eukaryoten Zellen, wie Elektroporation oder Lipofektion, haben zudem den entscheidenden Nachteil, daß die Behandlung die zu transportierende Substanz nur über die Plasmamembran bringt, die erste Barriere, die die Zelle von ihrer Umwelt abgrenzt. Es ist aber für die meisten in die regulatorischen Funktionen der Zelle eingreifenden Substanzen notwendig, aus dem Zytoplasma über die nukleäre Membran in den Zellkern zu gelangen. Diese Membran ist biophysikalisch grundsätzlich verschieden von der Plasmamembran, und Methoden wie Elektroporation oder Lipofektion überwinden diese Barriere nicht. Der Grund, warum die genannten Methoden dennoch bei einem Teil der Zellen z.B. zur Expression in die Zelle eingebrachter rekombinierter Nukleinsäurekonstrukte führt, ist die Tatsache, daß beim Vorgang der Zellteilung die nukleäre Membran offenbar durchlässig wird. Dies hat zur Konsequenz, daß mit Methoden wie Elektroporation oder Lipofektion nur Zellen transfiziert werden können, die sich teilen. Für viele sich langsam oder gar nicht teilende Zellarten, die im Rahmen möglicher gentherapeutischer Behandlungen interessant sind, wie Stammzellen des Immunsystems oder der Hämatopoiese, Muskelzellen, Zellen exokriner oder endokriner sekretorischer Organe, oder Neuronen und ihre Begleitzellen, kommen deshalb diese Methoden nicht in Frage. Die ebenfalls verbreitete, und in der Transfektionseffizienz sehr erfolgreiche Methode des viralen Transports von Erbmaterial hat den großen Nachteil, die transfizierten Zellen einer möglichen cytotoxischen Reaktion des Wirtsorganismus auszusetzen, was sie für gentherapeutische Ansätze nur sehr begrenzt in Frage kommen lassen dürfte.

Die Methode des ballistischen Transfers hat in der ex vivo Behandlung autologer oder allogener Patientenzellen Anwendung gefunden (Mahvi et. al.; Human Gene Therapy 7 (1996): 1535-43). Bei der Behandlung von Zellen im Zellverband allerdings, wie sie besonders für die onkotherapeutische Behandlung solider Tumore oder der massenhaften Infektionsprophylaxe durch genetische Vakzinierung vorteilhaft wäre, hat der Stand der Technik Nachteile: Die Methode des ballistischen Transfers bedient sich auf die Mikroprojektile adsorbierter DNA. Bei Transfektion von Haut oder anderen Gewebeverbänden ist die Eindringtiefe der DNA-Konstrukte niedriger als die Eindringtiefe der Projektile. Die DNA wird bald nach dem Auftreffen auf das Gewebe abgestreift. Es wird also nur jeweils die oberste, oder die oberen, in Richtung der Geschoßgarbe liegenden Zellschichten transfiziert, obwohl die Mikroprojektile z.T. viel tiefer in das Gewebe eindringen. Beim Transfizieren von solidem Tumorgewebe (Colonkarzinom, Rectumkarzinom, Nierenzellkarzinom und andere) hat man gefunden, daß bei geeigneter Einstellung der Parameter die Mikroprojektile eine Eindringtiefe von bis zu fünf Zellschichten in die Gewebeschnitte erreichten. Die transfizierten Zellen aber (sichtbar als fluoreszierende Zellen bei Transfektion mit einem rekombinanten Expressionskonstrukt, das das Gen für das grün fluoreszierende Protein aus *Aequorea Victoria* enthält) lagen alle nur in der obersten, der auftreffenden Garbe an Mikroprojektilen zugewandten Zellschicht des Gewebeschnitts. Eine festere Kopplung der DNA-Konstrukte an die Oberfläche der Mikroprojektile wäre wünschenswert, um das Abstreifen der zu transportierenden Substanz zu vermeiden. Erst so wird eine Anwendung gentherapeutischer Ansätze bei soliden Tumoren realistisch, da erst die Transfektion von Tumorschnitten in der Tiefe des Gewebes eine ausreichend hohe Anzahl behandelter Zellen ermöglicht. Gleichzeitig ist vorstellbar, daß eine Kombination verschiedener Kopplungsmethoden eine zeitlich verschobene Freisetzung mehrerer genetischer Informationen innerhalb der gleichen Zellpopulation ermöglicht. Für diese wie zahlreiche weitere Anwendungen wären Mikropartikel, die die zu transportierende Substanz bis in die getroffene Zelle bringen, wo die Substanz dann der Zelle zur Verfügung stünde, sehr wünschenswert.

Die US 5,584,807 (McCabe) beschreibt ein Instrument in Form einer gasdruckbetriebenen Pistole zum Einbringen von genetischem Material in biologisches Gewebe, wobei Goldpartikel als Trägermaterial für die genetische Information verwendet werden, ohne aber auf das genetische Material im besonderen einzugehen. Die US 5,580,859 und US 5,589,466 (Felgner) beschreibt ein Verfahren zum Einbringen von DNA in Säugerzellen im Rahmen der Gentherapie. Hierzu werden sogenannte nackte DNA-Sequenzen, die für physiologisch aktive Proteine, Peptide oder Polypeptide kodieren und mit einem Promotor versehen sind, direkt in Zellen injiziert. Die DNA wird in den Zellen exprimiert und dient als Vakzine.

Die WO 96/26270 (Rhone-Poulenc Rorer S.A.) beschreibt ein zirkuläres, doppelsträngiges (supercoiled) DNA Molekül, welches eine Expressionskassette enthält, kodierend für ein Gen und gesteuert über einen Promotor und einen Terminator. Auch dieses System wird in der Vakzinierung im Rahmen der Gentherapie eingesetzt.

Die EP 0 686 697 A2 (Soft Gene) betrifft eine Methode zur Anreicherung von Zellen, die durch ballistischen Transfer modifiziert wurden und beschreibt den technologischen Hintergrund und die damit zusammenhängende Problematik und ihre bisher bekannten Lösungen. Primär wird hierin jedoch das grundsätzliche Verfahren des ballistischen Transfers beschrieben. Eine geeignete Vorrichtung zur Durchführung dieses Verfahrens ist in der EP 0 732 395 A1 beschrieben.

Bei den ballistischen Partikeln handelt es sich (EP 0 686 697 A2) um Goldpartikel mit einem Durchmesser von entweder 1 µm oder 1,5 µm, die in Abhängigkeit vom Zelltyp ausgewählt werden. Die Goldpartikel sind mit superparamagnetischen Partikeln von ungefähr 30 nm Durchmesser beschichtet. Die superparamagnetischen Partikel stellen gleichzeitig eine geeignete Oberfläche für die Beschichtung mit Biomolekülen zur Verfügung. Die Verwendung von magnetischen Partikeln dient der Sortierung.

Bekannt sind ferner hantelförmige Nukleinsäurekonstrukte, die sich durch folgende Merkmale auszeichnen: Es handelt sich um kurze (10-50 Basenpaare doppelsträngige DNA) Nukleinsäurekonstrukte, die zur Strukturforschung hergestellt wurden oder als doppelsträngiges, nukleasebeständigeres Oligomer zum Abfangen sequenzspezifischer DNA-Liganden (Clusel et. al.; Nucleic Acids Res. 21 (1993): 3405-11; Lim et. al., Nucleic Acids Res. 25 (1997): 575-81)

Längere DNA-Moleküle, die zum Teil während ihres Replikationszyklus auch als Hantel vorliegen können, sind aus der Natur als mitochondriale Genome einiger Arten bekannt, so bei Wimperntierchen (*ciliatae*) und Hefen (Dinuel et. al., Molecular and Cell Biology, 13 (1993): 2315-23). Diese Moleküle sind etwa 50 kb groß und haben eine sehr komplexe genetische Struktur. Ebenfalls ist von *Vaccinia*-Virus eine geschlossen kovalente lineare Struktur bekannt.

Peptid-Nukleinsäureverknüpfungen mit Lokalisationssequenzen sind für kurze DNA-Oligomere bekannt. Morris et. al. (Nucleic Acids Res. 25 (1997): 2730-36) beschreiben die Kopplung von 18-meren bis 36-meren mit einem 27 Aminosäurereste enthaltenden Peptid, welches die Lokalisationssequenz aus SV40 enthält, sowie ein für die Fusion mit CD4-positiven Zellen verantwortliches Signalpeptid aus HIV-gp41.

Der Gebrauch von Peptidketten zur Überwindung der endosomalen Membran ist von mehreren Gruppen untersucht worden. So wurden die 23 N-terminalen Aminosäuren von Haemagglutinin über nicht-kovalente Wechselwirkungen an Expressionsplasmide adsorbiert, um diese Expressionsplasmide nach endosomaler Aufnahme der Komplexe in das Cytosol zu schleusen (Plank et. al., J. Biol. Chem. (1994) 269: 12918). Die kovalente Verknüpfung von antisense-Desoxyoligonukleotiden mit Haemagglutinin-Peptiden wird von Bongartz et al. (Nuc. Acids Res. (1994) 22: 4681) beschrieben.

Ausgehend von diesem Stand der Technik ist es Aufgabe der vorliegenden Erfindung, ein Expressionskonstrukt zu entwickeln, welches lediglich die für die Expression notwendige Information enthält und geeignete Mittel für den Transport in eine gentherapeutisch zu behandelnde Zelle zur Verfügung zu stellen.

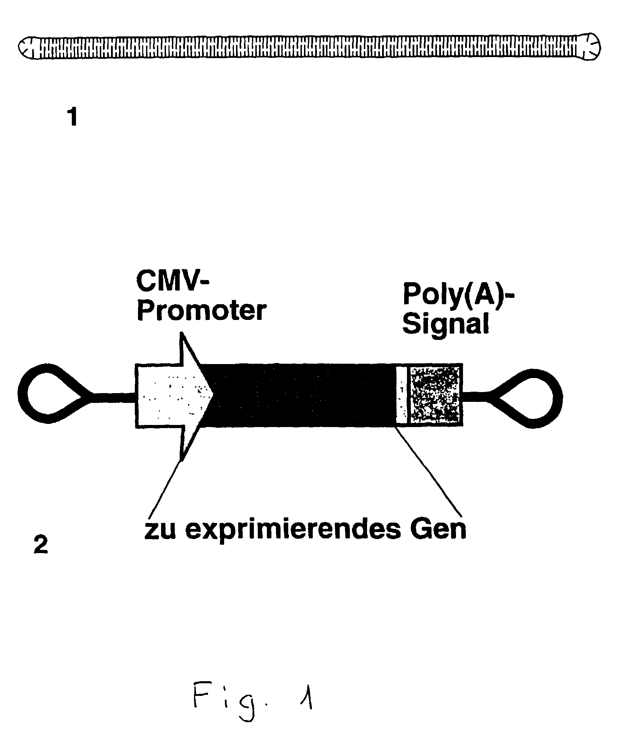

Gelöst wird diese Aufgabe durch die Merkmale des Anspruchs 1 und 13. Erfindungsgemäß werden zu transportierende doppelsträngige Expressionskonstrukte aus Desoxyribonukleinsäure so modifiziert, daß die beiden antiparallelen Stränge des Desoxyribonukleinsäurepolymers, welche die codierende Sequenz sowie die zu ihrer Expression notwendigen Promotor- und gegebenenfalls Terminationssequenzen enthalten, von je einer Schlaufe aus einzelsträngigen Desoxyribonukleotiden an beiden Enden derart kovalent verknüpft werden, daß ein durchgehend kovalent geschlossenes Molekül entsteht. Vorzugsweise ist diese Schlaufe drei bis sieben Nukleotide lang. In Figur 1 ist ein erfindungsgemäßes Konstrukt schematisch dargestellt. Derartige Expressionskonstrukte dienen zur Expression von MHC-I oder MHC-II präsentierbaren Peptiden, Cytokinen oder Komponenten der Regulation des Zellzyklus, oder zur Synthese regulativer RNA-Moleküle wie antisense-RNA, Ribozymen oder mRNA-editierende-RNA. Da die Nukleinsäure auf diese Weise kovalent geschlossen ist und keine freie OH-Enden zum Abbau zur Verfügung stellt, hat das Molekül eine sehr viel höhere Stabilität gegenüber intra- und interzellulären Nukleasen und damit eine längere Halbwertszeit im Körper bzw. der Zelle. Besonders schwer wiegt dieser Vorteil bei der Applikation *in vivo.*

Weiterhin können sich erfindungsgemäß in der genannten Schlaufe, die die Stränge miteinander verbindet, eine oder mehrere modifizierte Basen befinden, die chemische Funktionen enthalten, die die Kopplung mit einer festen Phase ermöglichen, vorzugsweise Amin-, Carbonsäure-, Thiol- oder Disulfidmodifikationen. Diese werden über bekannte Syntheseschritte mit korrespondierenden Carbonsäure-, Aldehyd-, Amin-, Thiol- oder anderen Funktionen oder direkt mit der Goldoberfläche eines Mikroprojektils zum ballistischen Transfer kovalent verbunden. Es ist vorstellbar, daß eine Kombination verschiedener Kopplungsmethoden eine zeitlich verschobene Freisetzung mehrerer genetischer Informationen innerhalb der gleichen Zellpopulation ermöglicht.

Neben dem Vorteil einer erleichterten chemischen Bindung an die Oberfläche des Mikroprojektils bietet die genannte Nukleinsäurekonstruktion auch einen weiteren Vorteil: Die heute zumeist zur Transfektion in der Gentherapie benutzten Nukleinsäurekonstrukte werden in Bakterien produziert und tragen, neben den im Zusammenhang mit der therapeutischen Anwendung relevanten Sequenzen, auch Sequenzen, die allein für die Vermehrung des Nukleinsäurekonstrukts in den Bakterien wichtig sind. Diese Sequenzen stellen ein nicht bekanntes Risiko für den zu behandelnden Patienten dar, da nicht bekannt ist, ob und gegebenenfalls wie sie sich im Organismus auswirken können. Solche Sequenzen, die nicht ausschließlich dem primären Ziel der Transfektion in den Zielzellen dienen, lassen sich vor der Transfektion aus in Bakterien vermehrten Nukleinsäuren durch Restriktionsendonukleaseverdau entfernen und durch kovalente Bindung von kurzen, gegebenenfalls chemisch modifizierten, Endstücken aus Desoxyribonukleinsäure ersetzen. Ebenfalls können erfindungsgemäß die in die Zelle zu transportierenden Desoxyribonukleinsäuren durch Polymerase-Kettenreaktion mit chemisch modifizierten Primern hergestellt werden, so daß die Produkte der Polymerase-Kettenreaktion die zur Bindung an das Mikroprojektil benötigte chemische Modifikation enthalten. Ein Vorteil des erfindungsgemäßen Konstruktionsprinzip gegenüber herkömmlichen Expressionsvektoren ist es daher, daß das entstehende Konstrukt nur die zur Expression des Zielgens notwendige Sequenz enthält.

Ein weiterer Aspekt der Erfindung ist, daß in der genannten Schleife aus einzelsträngigen Desoxyribonukleotiden an beiden Enden des Moleküls chemische Modifikationen so eingeführt werden können, daß sich nicht-Nukleinsäure-Liganden an das Nukleinsäure-Expressionskonstrukt kovalent koppeln lassen. So lassen sich etwa Peptide, die zur nukleären Lokalisation des Expressionskonstrukts führen, mit dem Konstrukt so verbinden, daß dieses auch nach Eindringen nur in das Cytosol einer Zelle von deren Translokationsapparat in nukleäre Kompartimente gebracht wird, wo das Expressionskonstrukt abgelesen werden kann. Die oben genannten Einschränkungen für manche Transfektionen wären damit aufgehoben. Ebenfalls zugänglich wäre die direkte Kopplung des Konstrukts an Peptid-, Glycopeptid- oder Kohlehydratliganden, welche das Eindringen des Konstrukts über zellspezifische Oberflächenrezeptoren ermöglichen könnten.

Insbesondere werden erfindungsgemäß zu transportierende doppelsträngige, antiparallele Expressionskonstrukte aus Desoxyribonukleinsäure so modifiziert, daß sie an den Enden der Doppelstränge je eine Disulfidbrücke enthalten, die die Stränge an beiden Enden über eine am 5'-Ende des einen und am 3'-Ende des anderen Stranges kovalent gebundene Alkylgruppe miteinander verknüpft.

Als Promotor für die Transkriptionskontrolle des Expressionsplasmids eignet sich prinzipiell jede eukaryote Promotorsequenz. Besonders vorteilhaft sind kurze Promotoren mit hoher Transkriptionsrate, welche in einer Vielzahl von Zellen transkribiert werden können, wie z.B. der "immediate early promotor" aus Cytomegalovirus (CMV-Promotor). Für die Transkription von Genen, welche für RNA kodieren, sind RNA-Polymerase III-abhängige Promotoren wie der 7SK-Promotor oder der U6-Promotor vorteilhaft. Solche Promotorsequenzen können *in vivo* die Expression kurzer antisense-RNAs, von Ribozymen und artifizieller mRNAs bewirken. RNA-Polymerase III erzeugt weitaus höhere Anzahlen an Kopien von RNA als Polymerase II und hat - was besonders vorteilhaft ist - ein exaktes Terminationssignal. Die genannten Promotorsequenzen zeichnen sich durch ihre Kürze aus, was die entsprechenden Hantel-Expressionskonstrukte klein macht, was wiederum deren Eintritt in den Zellkern bevorteilt.

Die Erfindung betrifft auch den Transport von Nukleinsäuren in Zellen. Nukleinsäuren werden in Zellen erfindungsgemäß dadurch eingebracht, daß die Nukleinsäuren an die Oberfläche des Mikroprojektils, das die Nukleinsäuren in die Zellen bringt, über Adsorption, kovalente oder ionische Wechselwirkungen so gebunden werden, daß sie beim Durchtreten des Mikroprojektils durch Bindegewebe, die Zellen umgebende Flüssigkeit oder Zellschichten nicht oder nicht vollständig desorbiert werden, sondern an die Mikroprojektile gebunden bleiben, bis sie mit diesen in den Zielzellen zur Ruhe kommen. Dies hat den Vorteil, daß die Substanz dann je nach Bindungsart entweder der Zelle in gebundenem Zustand zur Verfügung steht, oder aber in einem, im Vergleich zur Eintrittszeit in die Zelle langsameren, Prozeß desorbiert wird und in diesem desorbierten Zustand der Zelle zur Verfügung steht.

Grundsätzlich ist die Bindung von Thiolen oder Disulfiden an Goldoberflachen gut untersucht und beschrieben (G. M. Whitesides et al., Langmuir 10 (1994): 1825-1831). Die zu transportierenden Nukleinsäuren werden jedoch vorzugsweise an Mikroprojektile aus Gold gebunden, indem die Nukleinsäuren zuerst durch kovalente Modifikation mit handelsüblichen Reagenzien mit Thiol- oder Disulfidgruppen ausgestattet werden, und dann über die Thiol- oder Disulfidgruppen adsorbiert, oder durch anodische Oxidation der Thiol- oder Disulfidfunktion unter Verwendung des Gold-Mikroprojektils als Anode kovalent an das Mikroprojektil gebunden werden. Insbesondere wird erfindungsgemäß die zu transportierende Substanz durch Verknüpfung über Schwefel-Gold-Bindungen oder Disulfidbindungen an das Mikroprojektil gebunden. Bei Verwendung von Mikroprojektilen aus Gold wird die zu transportierende Substanz, wenn sie von sich aus keine Thiol- oder Disulfidgruppen, die bindungsfähig waren, enthält, durch kovalente Verknüpfung mit Thiolgruppen oder Disulfidbrücken enthaltenen Molekülen modifiziert. Sie wird dann durch Chemisorbtion an die Goldoberfläche des Mikroprojektils gebunden. Die entstehende Gold-Schwefelbindung ist stark genug, das zu transportierende Molekül auch durch mehrere Zellschichten hindurch mitzureißen.

Da in der Zelle Moleküle vorhanden sind, die Thiolgruppen aufweisen, vor allem das ubiquitäre Glutathion, kommt es in einer Gleichgewichtsreaktion der Thiolgruppen an der Goldoberfläche zu einer langsamen Desorbtion des chemisorbierten Moleküls von der Oberfläche des Mikroprojektils. Somit steht die transportierte Substanz der Zelle nach der Desorbtion frei zur Verfügung. Weiterhin kann erfindungsgemäß bei Verwendung von Mikroprojektilen aus Gold das zu transportierende Molekül, das Thiol- oder Disulfidbrücken aufweist, durch anodische Oxidation der Thiol- oder Disulfidfunktion unter Verwendung des Gold-Mikroprojektils als Anode kovalent an das Mikroprojektil gebunden werden.

Erfindungsgemäß ist auch vorgesehen, daß bei Verwendung von Mikroprojektilen aus oxidischer Keramik, Glaskeramik oder Glas das zu transportierende Molekül über Ester-, Amid-, Aldimin-, Ketal-, Acetal- oder Etherbindungen oder sonstige Funktionalitäten, die dem organischen Chemiker zur Bindung von Molekülen an feste Oberflächen bekannt sind, gebunden werden. Die zahlreichen zur Modifikation von Siliciumoxidphasen zur Verfügung stehenden Silanreagenzien bieten sich hier an.

Die Erfindung dient dem Einsatz in der *ex vivo* Gentherapie. Bevorzugt werden Interleukin-7 (IL-7) und Interleukin-12 (IL-12) Proteine oder deren Untereinheiten exprimiert, sowie Interleukine, Granulocyten-Makrophagen-Koloniestimulierender Faktor (GM-CSF), Oberflächenantigene und Liganden immunkontrollierender oder-stimulierender Lymphozytenantigene wie CD40, B7-1 und B7-2, Proteine des MHC-Komplexes I oder II sowie beta-2 Mikroglobulin, Interferon-Konsensussequenz-bindendes Protein ICSBP, CIITA, Flt3 sowie ganze Proteine oder Fragmente dieser von präsentierbaren Epitopen aus tumorspezifisch exprimierten mutierten oder nichtmutierten Proteinen wie Ki-RAS-Fragmente, p16 und p53, oder bcr-abl Produkt. Bevorzugt ist zum einen der Einsatz von Mikropartikeln, die alle mit demselben Konstrukt markiert sind, zum anderen aber auch Cocktails (Mischungen) aus Mikroprojektilen, die mit unterschiedlichen Konstrukten verknüpft sind, das heißt mit Konstrukten, die für unterschiedliche Expressionskonstrukte kodieren, oder aber auch Cocktails aus Mikroprojektilen, die jeweils mit mehreren, ggf. unterschiedlichen Konstrukten verknüpft sind.

Das erfindungsgemäße Desoxyribonuklinsäurekonstrukt dient bevorzugt auch als Impfstoff zur Behandlung von Infektionskrankheiten an Mensch und Tier, beispielsweise Malaria und Grippe.

Weitere vorteilhafte Maßnahmen sind in den übrigen Unteransprüchen enthalten. Die Erfindung ist in den anliegenden Zeichnungen dargestellt und wird nachfolgend anhand von Beispielen näher beschrieben. Es zeigt:
- **Figur 1**: eine schematische Darstellung des Konstruktkonzeptes, worin Fig. 1.1 den Aufbau der kovalent geschlossenen Phosphat-Zuckerkette zeigt; die dargestellte Zahl an Basenpaaren entspricht nicht notwendigerweise der Länge der Konstrukte, sondern dient nur der prinzipiellen Verdeutlichung; Fig. 1.2 zeigt den funktionellen Aufbau eines von mehreren möglichen Expressionskonstrukten;
- **Figur 2**: zeigt den schematischen Ablauf der Konstruktdarstellung.

### Beispiel 1 [Synthese der Expressionskonstrukte]:

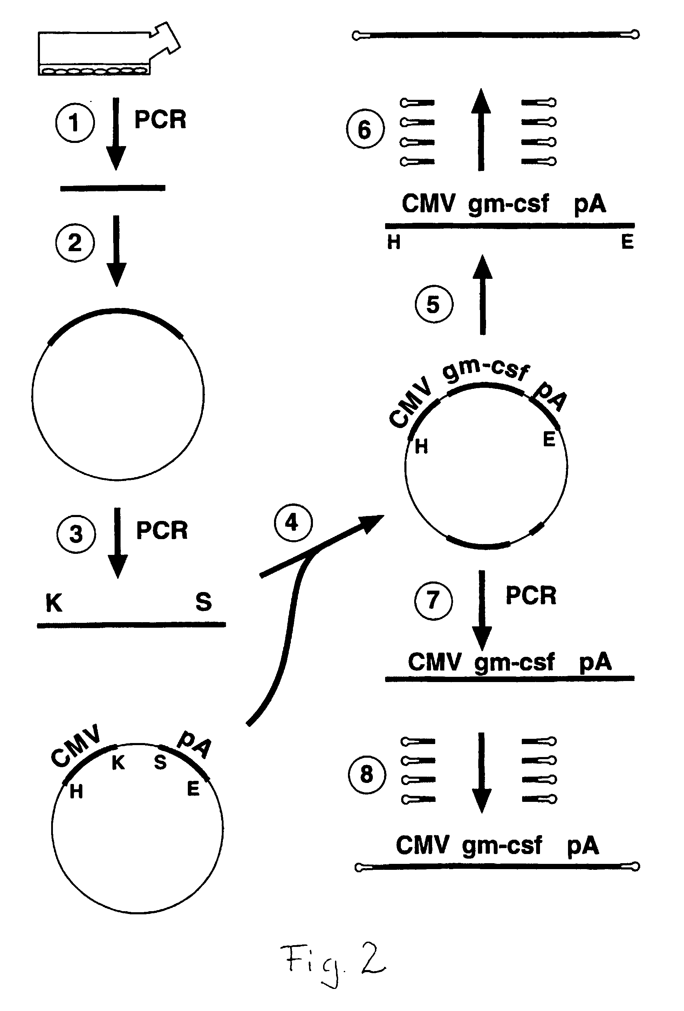

Das grundlegende Konstruktionsprinzip wird in Fig. 2 schematisch wiedergegeben und sieht wie folgt aus:

### 1.1 Synthese aus Vektor:

Das zu exprimierende Gen, z.B. Granulozyten-Makrophagenstimulierender Faktor gm-csf, wird aus cDNA mittels geeigneter Primer mittels PCR amplifiziert (Fig. 2 (1)) und in einen geeigneten Plasmidvektor rekombiniert (Fig. 2 (2)). Nach Sequenzierung und Bestätigung der Zielsequenz wird die zu exprimierende Sequenz aus dem genannten Plasmidvektor mittels zweier Primer (Oligodesoxynukleotide, welche an der 5'-Seite ihrer Sequenz Erkennungsstellen für Restriktionsendonuleasen tragen) amplifiziert (Fig. 2 (3)). Das entstehende Amplifikationsprodukt wird mit den genannten Endonukleasen, für welche eine Erkennungssequenz auf den genannten Primern vorhanden war, verdaut. Nach Isolierung des amplifizierten Fragments aus Agarosegel wird dieses in ein Expressionsplasmid rekombiniert, welches in Bakterien vermehrbar ist, und in welchem das zu exprimierende Gen in der gewünschten Orientierung im Kontext der auf dem Expressionsplasmid befindlichen expressionssteuernden Sequenzen angeordnet ist (Fig. 2 (4)).

Besagtes Expressionsplasmid wird in Bakterien vermehrt und nach bekannten Methoden isoliert. Nach Verdau mit Restriktionsendonukleasen, deren Erkennungssequenzen die Sequenz flankieren, welche das hantelförmige Expressionskonstrukt tragen soll, werden die Restriktionsfragmente durch geeignete Methoden mit AnionenAustauschchromatographie getrennt voneinander isoliert (Fig. 2 (5)) und anschliessend mit haarnadelförmig selbsthybridisierenden Oligodesoxynukleotiden (kurze, durch automatisierte chemische DNA-Synthese hergestellte DNA-Moleküle, die aufgrund ihrer Selbstkomplementarität Stamm-Schlaufenstrukturen bilden können; diese Moleküle formen später die kovalent geschlossenen Enden der hantelförmigen DNA-Moleküle) welche einen auf den durch Restriktionsverdau entstandenen Überhang des Konstruktes passenden Überhang ihrer Doppelstrangstruktur aufweisen, ligiert (Fig. 2 (6)). Nach Abtrennen der überschüssigen Haarnadel-Desoxyoligonukleotide mittels AnionenAustauschchromatographie liegen die die Erfindung ausmachenden Konstrukte vor.

### 1.2 Synthese aus PCR-Produkt

Alternativ wird das Konstrukt direkt mittels Polymerase-Kettenreaktion vermehrt, wobei die Primeroligodesoxynukleotide an der 5'-Seite ihrer Sequenz Erkennungsstellen für Restriktionsendonuleasen tragen (Fig. 2 (7)). Nach ionentausch-chromatographischem Abtrennen der Primer wird das entstandene Amplifikationsprodukt mit den Endonukleasen, für welche eine Erkennungssequenz auf den genannten Primern vorhanden war, verdaut. Nach erneutem Abtrennen der kleineren Restriktionsfragmente wird das Konstrukt mit kurzen, haarnadelförmig selbsthybridisierenden Oligodesoxynukleotiden, welche einen auf den durch Restriktionsverdau entstandenen Überhang des Konstruktes passenden Überhang ihrer Doppelstrangstruktur aufweisen, ligiert (Fig. 2 (8)). Nach Abtrennen der überschüssigen Haarnadel-desoxyoligonukleotide mittels Anionen-Austauschchromatographie liegen die die Erfindung ausmachenden Konstrukte vor.

Ein Expressionskonstrukt, welches aus der Sequenz für gm-csf unter Kontrolle des "early immediate promotor" aus CMV und der Polyadenylierungssequenz aus SV40 besteht, wurde aus dem Plasmid mtv-gmcsf durch vollständigen Verdau mit EcoRI und Hind III ausgeschnitten. Das kleinere Fragment (1290 bp) wurde mit Anionenaustauschchromatographie (stat.Phase: Merck Fractogel EMD-DMAE; 25 mM Tris/HCl pH 8; 0-1M NaCl) isoliert und nach Konzentration und Entsalzung mit einem 200-fachen molaren Überschuss an 5'-phosphorylierten Haarnadel-Desoxyoligoribonukleotiden AATTCGGCCGGCCGTTTTCGGCCGGCCG und AGCTTGGCCGGCCGTTTTCGGCCGGCCA (TIB-Molbiol, Berlin) in Anwesenheit von 25 u/ml T4-DNA-Ligase über Nacht bei Raumtemperatur inkubiert. Die Reaktion wurde durch Erhitzen auf 60°C gestoppt. Das mit dem Desoxyoligoribonukleotid ligierte Konstrukt wurde durch Anionenaustauschchromatographie von dem überschüssigen Desoxyoligoribonukleotid abgetrennt, durch Ethanolfällung konzentriert, in Wasser aufgenommen und nach bekannten Verfahren im ballistischen Transfer auf sterile primäre Coloncarcinom-Zellen angewendet.

### Beispiel 2 [in vivo Expression]:

### Ballistischer Transfer von gm-csf auf K562:

30 µl einer Suspension von Goldpartikeln (1.6 µm Durchmesser, bezogen von Bio-Rad, Hercules, CA, USA, Konzentration der Suspension: 30 mg/ml) werden auf eine Makrocarrier-Polymerplatte (Fa. Bio-Rad) aufpippettiert. Man wartet, bis sich das Gold abgesetzt hat, und zieht den Überstand vorsichtig ab. Auf die benetzte Fläche wird 30 µl einer 1+3 Mischung aus einer Suspension kolloidaler Magnetpartikel (mittlerer Durchmesser: 65 nm; Miltenyi GmbH, Bergisch-Gladbach, Deutschland; eingesetzt, wie vom Hersteller geliefert; Konz. unbekannt) und gmcsf-Expressions-Hantelkonstrukt (siehe Bsp. 1) pipettiert. Man wirbelt das abgesetzte Gold auf und läßt das Gemisch sich absetzen. Man zieht die überstehende Flüssigkeit ab und läßt die Goldpartikel antrocknen. Auf eine Petrischale (3,5 cm) werden 300 µl Polylysin in der Mitte aufbebracht, 30 min stehen gelassen und mit PBS-Medium abgespült. Zwischen 100.000 und 200.000 Zellen (Erythroleukämiezellinie K 562) werden in 300 µl RPMI-Medium (10% FKS) auf die polylysinbeschichtete Fläche der Petrischale gebracht und 10 min stehen gelassen. Man überschichtet mit 2 ml RPMI-Medium (10% FKS) und läßt 1-2 h im Brutschrank inkubieren.

Der ballistische Transfer wird an einer Biolistic PDS 1000/He (Bio-Rad, Hercules, CA, USA) nach Vorschrift des Herstellers des Apparats durchgeführt. Die verwendete Berstscheibe entspricht einem Sollbruchdruck von 1.100 psi. Der Druck der Vakuumzelle beträgt 508 mm Quecksilbersäule (20 inches Hg). Magnetische Separation erfolgt wie veröffentlicht (EP 0 732 395 A1); Erfolgskontrolle erfolgt durch gm-csf-ELISA.

### Beispiel 3 [Beispiel für Herstellung eines Expressionskonstruktes mit nukleärer Lokalisationssequenz]:

Ein Expressionskonstrukt, welches aus der Sequenz für das grün fluoreszierende Protein EGFP (Clonetech) unter Kontrolle des "early immediate promotor" aus CMV und der Polyadenylierungssequenz aus SV40 besteht, wurde aus dem Plasmid mtv-egfp durch vollständigen Verdau mit EcoRI und HindIII ausgeschnitten. Das kleinere Fragment (1601 bp) wurde mit Anionenaustauschchromatographie (stat.Phase: Merck Fractogel EMD-DMAE; 25 mM Tris/HCl pH 8; 0-1M NaCl) isoliert und nach Konzentration und Entsalzung mit einem 200-fachen molaren Überschuss an 5'-phosphorylierten Haarnadel-Desoxyoligoribonukleotiden AATTCGGCCGGCCGTXTCGGCCGGCCG und AGCTTGGCCGGCCGTXTCGGCCGGCCA in Anwesenheit von 25 u/ml T4-DNA-Ligase über Nacht bei Raumtemperatur inkubiert. (X bezeichnet die Peptidmodifikation: Amino-Uracil mit über X-Kopplung verbundenem Peptid (TIB-Molbiol, Berlin)). Die Reaktion wurde durch Erhitzen auf 60°C gestoppt. Das mit dem thiolmodifizierten Desoxyoligoribonukleotid ligierte Konstrukt wurde durch Anionenaustauschchromatographie von dem überschüssigen Desoxyoligoribonukleotid abgetrennt, durch Ethanolfällung konzentriert und in Wasser aufgenommen. 1 µg des thiolmodifizierten Konstruktes wurde mit 1 mg Mikroprojektilen (Gold sphärisch, mittlerer Durchmesser 1 µm, Bio-Rad, Hercules, CA) in Wasser über Nacht bei Raumtemperatur inkubiert. Die Goldpartikel wurden zwei Mal mit Wasser gewaschen und nach bekannten Verfahren im ballistischen Transfer auf festwachsende Keratinozyten angewendet.

### Beispiel 4 [Synthese der Nukleinsäure-modifizierten GoldPartikel]:

Ein Expressionskonstrukt, welches aus der Sequenz für gm-csf unter Kontrolle des "early immediate promotor" aus CMV und der Polyadenylierungssequenz aus SV40 besteht, wurde aus dem Plasmid mtv-gmcsf durch vollständigen Verdau mit EcoRI und HindIII ausgeschnitten. Das kleinere Fragment (1290 bp) wurde mit Anionenaustauschchromatographie (stat. Phase: Merck Fractogel EMD-DMAE; 25 mM Tris/HCl pH 8; 0-1M NaCl) isoliert und nach Konzentration und Entsalzung mit einem 200-fachen molaren Überschuss an 5'-phosphorylierten Haarnadel-Desoxyoligoribonukleotiden AATTCGGCCGGCCGTXTCGGCCGGCCG und AGCTTGGCCGGCCGTXTCGGCCGGCCA (X bezeichnet den Thiol-Modifier C6 S-S (TIB-Molbiol, Berlin)) in Anwesenheit von 25 u/ml T4-DNA-Ligase über Nacht bei Raumtemperatur inkubiert. Die Reaktion wurde durch Erhitzen auf 60°C gestoppt. Das mit dem thiolmodifizierten Desoxyoligoribonukleotid ligierte Konstrukt wurde durch Anionenaustauschchromatographie von dem überschüssigen thiolmodifizierten Desoxyoligoribonukleotid abgetrennt, in Wasser aufgenommen 1 µg des thiolmodifizierten Konstruktes wurde mit 1 mg Mikroprojektilen (Gold sphärisch, mittlerer Durchmesser 1 µm, Bio-Rad, Hercules, CA) in Wasser über Nacht bei Raumtemperatur inkubiert. Die Goldpartikel wurden zwei Mal mit Wasser gewaschen und im ballistischen Transfer auf sterile primäre Coloncarcinom-Zellen angewendet (siehe Bsp. 5).

### Beispiel 5 [Ballistischer Transfer auf solides Tumorgewebe]:

Steriles primäres Coloncarcinomgewebe wurde chirurgisch entnommen und auf Eis gekühlt. Nekrotische und Bindegewebsteile werden, soweit möglich, entfernt. Stücke von etwa 1 cm² Oberfläche werden herausgeschnitten, in eiskaltem PBS gewaschen und auf dem Probenhalter eines Gewebeschneiders (vibratome 1000 sectioning system; TPI, St. Louis, Missouri) mit Gewebeklebstoff fixiert. Der Tumor wird in 500 µm dicke Scheiben zerschnitten. Die Scheiben werden in eiskaltem PBS gelagert und werden so schnell wie möglich transfiziert. 30 µl einer Suspension von gm-csf-Expressionskonstrukt-beschichteten Goldpartikeln und kolloidaler Magnetpartikel (mittlerer Durchmesser: 65 nm; Miltenyi GmbH, Bergisch-Gladbach) werden auf eine Makrocarrier-Polymerplatte (Fa. Bio-Rad) aufpippettiert. Man wartet, bis sich das Gold abgesetzt hat, zieht den Überstand vorsichtig ab und läßt die Goldpartikel antrocknen. Der Vorgang des ballistischen Transfers ist mit dem in Beispiel 2 beschriebenen Vorgehen identisch. Es werden beide Seiten der Scheibe beschossen. Nach der Transfektion wird die Scheibe zwei Mal durch ein Zellsieb passiert, und die Zellen werden separiert wie beschrieben.

Magnetische Separation erfolgt wie veröffentlicht (EP 0 732 395 A1); Erfolgskontrolle erfolgt durch gm-csf-ELISA.

### Beispiel 6 [Synthese der Nukleinsäure-modifizierten Aluminium-Partikel]:

5 µg des Expressionskonstruktes, welches aus der Sequenz für das grün fluoreszierende Protein EGFP (Clonetech) unter Kontrolle des "early immediate promotor" aus CMV und der Polyadenylierungssequenz aus SV40 besteht, wurde aus dem Plasmid mtv-egfp durch vollständigen Verdau mit EcoRI und HindIII ausgeschnitten. Das kleinere Fragment (1601 bp) wurde mit Anionenaustauschchromatographie (stat.Phase: Merck Fractogel EMD-DMAE; 25 mM Tris/HCl pH 8; 0-1M NaCl) isoliert und nach Konzentration und Entsalzung mit einem 200-fachen molaren Überschuss an 5'-phosphorylierten Haarnadel-Desoxyoligoribonukleotiden AATTCGGCCGGCCGTYTCGGCCGGCCG und AGCTTGGCCGGCCGTYTCGGCCGGCCA (Y bezeichnet das Carbonsäure-modifizierte Thymidindesoxynukleotid (TIB Molbiol, Berlin) in Anwesenheit von 25 u/ml T4-DNA-Ligase über Nacht bei Raumtemperatur inkubiert.

1 g Aluminiumoxidpartikel (mittlerer Durchmesser 1,0 µm) wurden in einer Lösung von Triethoxyaminopropylsilan in Toluol (2%) über Nacht am Rückfluß gekocht. Der Feststoff wird abfiltriert, mit Toluol, anschließend Ethanol gewaschen, getrocknet und erneut gemahlen. 5 mg des erzeugten aminomodifizierten Aluminiumoxids werden in 100 ml wäßrigem Carbonatpuffer (pH 8,0) mit 4 µg des Carbonsäuremodifizierten Konstruktes in Anwesenheit von 50 mM 1-Ethyl-3-(3-Dimethylaminopropyl)carbodiimid und 50 mM N-Hydroxysuccinimid 2 h bei Raumtemperatur umgesetzt. Die erzeugten nukleinsäuregekoppelten Mikropartikel können durch Beschleunigung auf Zellen in einer geeigneten Apparatur, wie z.B. in der DE 195 10 696 und EP 0 732 395 A1 beschrieben, zum Eindringen in die Zellen gebracht werden, wodurch die auf dem transportierten Plasmid enthaltene Information der Zelle zugänglich gemacht wird.

## Patentansprüche

1. Desoxyribonukleinsaurekonstrukt zur Transkription von RNA-Molekülen, gekennzeichnet durch einen zirkulären Strang Desoxyribonukleinsäure mit einer teilweise zueinander komplementären, antiparallelen Basensequenz, so daß ein hantelförmiges Konstrukt entsteht,
- wobei der zueinander komplementäre, antiparallele Basenabschnitt im wesentlichen aus einem Promotor, einem kodierendem Bereich und entweder einem Poly(A)-Additionssignal oder einer anderen RNA-stabilisierenden Sequenz besteht,
- und der nicht komplementäre Basenabschnitt zwei Schlaufen einzelsträngiger Desoxyribonukleinsäure bildet, die jeweils das 5'- und 3'-Ende des komplementären, antiparallelen Basenabschnitts kovalent miteinander verbinden.

2. Desoxyribonukleinsäurekonstrukt nach Anspruch 1, dadurch gekennzeichnet, daß die Schlaufen aus drei bis sieben Nukleotiden gebildet werden, von denen eines oder mehrere durch eine oder mehrere alkylische Carbonsäure-, Amin-, Thiol- oder Aldehydfunktionen chemisch modifiziert sind.

3. Desoxyribonukleinsäurekonstrukt nach Anspruch 2, dadurch gekennzeichnet, daß mit den chemisch modifizierten Nukleotiden ein Peptid kovalent verknüpft ist, welches zum gerichteten Transport des Konstrukts in den Zellkern führt.

4. Desoxyribonukleinsäurekonstrukt nach Anspruch 2, dadurch gekennzeichnet, daß mit den chemisch modifizierten Nukleotiden ein Peptid kovalent verknüpft ist, welches das Verlassen des Konstruktes aus dem Endosom in das Cytosol ermöglicht.

5. Desoxyribonukleinsäurekonstrukt nach Anspruch 1, dadurch gekennzeichnet, daß ein 7-SK RNA-Promotor verwendet wird.

6. Desoxyribonukleinsäurekonstrukt nach Anspruch 1, dadurch gekennzeichnet, daß ein CMV-Promotor verwendet wird.

7. Desoxyribonukleinsäurekonstrukt nach wenigstens einem der Ansprüche 1 bis 6, kodierend für Interleukin-7.

8. Desoxyribonukleinsäurekonstrukt nach wenigstens einem der Ansprüche 1 bis 6, kodierend für Interleukin-12 oder eine oder mehrere Interleukin-12-Untereinheiten.

9. Desoxyribonukleinsäurekonstrukt nach wenigstens einem der Ansprüche 1 bis 6, kodierend für GM-CSF.

10. Desoxyribonukleinsäurekonstrukt nach wenigstens einem der Ansprüche 1 bis 6, kodierend für p16 oder p53 Protein oder deren Fragmente.

11. Desoxyribonukleinsäurekonstrukt nach wenigstens einem der Ansprüche 1 bis 6, kodierend für Aminosäuren-Fragmente aus mutierten Ki-RAS, mutiertem p53 oder bcr-abl Translokationsprodukt mit einer Sequenzlänge von 10-100 Aminosäuren.

12. Mikroprojektil zum ballistischen Transfer von Desoxyribonukleinsäurekonstrukten in Zellen gemäß einem oder mehreren der Ansprüche 1 bis 11, dadurch gekennzeichnet, daß die zu transportierende Substanz durch Adsorptionswechselwirkungen oder kovalente oder ionische Bindungen derart an das Mikroprojektil gebunden wird, daß die zu transportierende Substanz beim Durchtreten des Mikroprojektils durch Bindegewebe, die Zellen umgebende Flüssigkeit oder Zellschichten nicht oder nicht vollständig desorbiert wird, sondern an das Mikroprojektil gebunden bleibt, bis die zu transportierende Substanz mit dem Mikroprojektil in der Zielzelle zur Ruhe kommt.

13. Mikroprojektil nach Anspruch 12, dadurch gekennzeichnet, daß das Mikroprojektil aus einem der Materialien Gold, mikrokristallines Oberflächengold, Oxidkeramik, Glaskeramik oder Glas besteht und die zu transportierende Nukleinsäure über Thiol- oder Disulfidfunktion, Ester-, Amid-, Aldimin-, Ketal-, Acetal-, oder Etherbindungen kovalent mit dem Mikroprojektil verknüpft ist.

14. Mikroprojektil nach Anspruch 12 oder 13, dadurch gekennzeichnet, daß besagtes Mikroprojektil aus einem elektrisch leitfähigen Material besteht und die Nukleinsäure durch elektrochemische Reaktion einer mit der Nukleinsäure durch kovalente Bindung verbundenen Thiol- oder Disulfidfunktion unter Verwendung des Mikroprojektils als Elektrode kovalent an das Mikroprojektil gebunden wird.

15. Mikroprojektil nach Anspruch 12 bis 14, dadurch gekennzeichnet, daß das Mikroprojektil eine Korngröße von 0,3 bis 3,0 µm aufweist.

16. Verwendung der Desoxyribonukleinsäurekonstrukte gemäß einem oder mehreren der Ansprüche 1 bis 11 in der *ex vivo* Gentherapie.

17. Verwendung der Mikroprojektile gemäß einem oder mehreren der Ansprüche 12 bis 15 in der *ex vivo* Gentherapie.

18. Verwendung der Desoxyribonukleinsäurekonstrukte gemäß einem oder mehreren der Ansprüche 1 bis 6 als Impfstoff zur Behandlung von Infektionskrankheiten an Mensch und Tier.

## Claims

1. Deoxyribonucleic acid construct for transcription of RNA-molecules, characterized by a circular strand of deoxyribonucleic acid comprising a partly complementary, antiparallel base sequence, so that a dumbbell-shaped construct is formed,
- in which the complementary, antiparallel base sequence in the essential comprises a promotor sequence, a coding sequence and a polyadenylation signal or another RNA-stabilizing signal,
- and the non-complementary sequence comprises two loops of single-stranded deoxyribonucleic acid, which covalently join the 5'- and 3'- ends of the complementary, antiparallel strands.

2. Deoxyribonucleic acid construct according to claim 1, characterized by that said loops consisting of three to seven nucleotides, and in which one or several of said nucleotides are covalently modified by carboxylic acid-, amine-, thiole- or aldehyde functionalities.

3. Deoxyribonucleic acid construct according to claim 2, characterized by that said chemically modified nucleotides is being linked to a peptide leading to the directed transport of the construct into the nucleus.

4. Deoxyribonucleic acid construct according to claim 2, characterized by that said chemically modified nucleotides is being linked to a peptide enabling liberation of the construct from the endosome.

5. Deoxyribonucleic acid construct according to claim 1, characterized by using a 7SK promoter as said promoter.

6. Deoxyribonucleic acid construct according to claim 1, characterized by using a CMV promoter as said promoter.

7. Deoxyribonucleic acid construct according to at least one of the claims a to 6, coding for interleukine-7.

8. Deoxyribonucleic acid construct according to at least one of the claims 1 to 6, coding for interleukine-12 or one or several of its constituting subunits.

9. Deoxyribonucleic acid construct according to at least one of the claims 1 to 6, coding for gm-csf.

10. Deoxyribonucleic acid construct according to at least one of the claims 1 to 6, coding for p16 or p53 protein or fragments thereof.

11. Deoxyribonucleic acid construct according to at least one of the claims 1 to 6, coding for peptide fragments of mutated ki-ras, mutated p53 or bcr-abl translocation product with a length of between 10 and 100 amino acids.

12. Micro projectile for ballistic transfer of deoxyribonucleic acid constructs into cells according to one or several of claims 1 to 11, in which the substance to be transported is linked by adsorption or covalent or ionic binding in such a way to said micro projectile that the substance to be transported upon passage of the micro particle through connective tissue, the extra cellular liquid or cell layers is not or not completely desorbed, but remains bound to said micro projectile until the substance to be transported rests along with said micro projectile in the target cell.

13. Micro projectile according to claim 12, characterized by that its material being gold, micro crystalline gold, oxide ceramic, glass ceramic or glass and said nucleic acid to be transported is bound covalently by thiole- or disulfide moieties, ester-, amide-, aldimine-, ketale- or acetale- or ether functionalities to said micro projectile.

14. Micro projectile according to claim 12 or 13, characterized by that said micro projectile being made out of an electrically conducive material and said nucleic acid being linked to said micro projectile by electrochemically coupling of disulfide or thiole moieties, employing the micro projectile as electrode.

15. Micro projectile according to claim 12 to 14, characterized by that said micro projectile being of the size of 0,3 µm to 3 µm.

16. Use of a nucleic acid construct according to one or more of the claims 1 to 11 in *ex-vivo* gene therapy.

17. Use of a micro projectile according to one or more of the claims 12 to 15 in ex-vivo gene therapy.

18. Use of said deoxyribonucleic acid constructs according to one or several of the claims 1 to 6 for producing a vaccine for prevention of infectious disease in humans or animals.

## Revendications

1. Construction d'acide désoxyribonucléique destinée à la transcription de molécules d'A.R.N., caractérisée par un brin circulaire d'acide désoxyribonucléique avec une séquence de bases antiparallèle partiellement complémentaire, ce qui produit une construction en forme d'haltère.
- la région antiparallèle complémentaire des bases étant constituée pour l'essentiel d'un promoteur, d'une partie codante et soit d'un signal polyadditionnel (A) soit d'une autre séquence stabilisant l'A.R.,
- et la région non complémentaire des bases formant deux boucles d'A.D.N. à simple brin, qui établissent une liaison covalente entre les extrémités 5' et 3' de la région complémentaire et antiparallèle des bases.

2. Construction d'acide désoxyribonucléique selon la revendication n° 1, caractérisée par le fait que les boucles sont constituées de trois à sept nucléotides dont un ou plusieurs ont été modifiés chimiquement par une ou plusieurs fonctions aldéhyde, thiol, amine ou carboxyle alkyles.

3. Construction d'acide désoxyribonucléique selon la revendication n° 2, caractérisée par la liaison covalente avec les nucléotides modifiés chimiquement d'un peptide qui conduit au transport ciblé de la construction dans le noyau de la cellule.

4. Construction d'acide désoxyribonucléique selon la revendication n° 2, caractérisée par la liaison covalente avec les nucléotides modifiés chimiquement d'un peptide qui permet à la construction de passer de l'endosome dans le cytosol.

5. Construction d'acide désoxyribonucléique selon la revendication n° 1, caractérisée par l'utilisation d'un promoteur RNA 7 SK.

6. Construction d'acide désoxyribonucléique selon la revendication n° 1, caractérisée par l'utilisation d'un promoteur CMV.

7. Construction d'acide désoxyribonucléique selon au moins l'une des revendications 1 à 6, codante pour l'interleukine-7.

8. Construction d'acide désoxyribonucléique selon au moins l'une des revendications 1 à 6, codante pour l'interleukine-12 ou une ou plusieurs sous-unités d'interleukine-12.

9. Construction d'acide désoxyribonucléique selon au moins l'une des revendications 1 à 6, codante pour GM-CSF.

10. Construction d'acide désoxyribonucléique selon au moins l'une des revendications 1 à 6, codante pour la protéine p16 ou p53 ou ses fragments.

11. Construction d'acide désoxyribonucléique selon au moins l'une des revendications 1 à 6, codante pour les fragments d'acides aminés de Ki-RAS mutés, de p53 mutée ou de produit de translocation bcr-abl avec une longueur de séquence de 10 à 100 acides aminés.

12. Microprojectile pour transfert balistique de constructions d'acide désoxyribonucléique dans les cellules selon une ou plusieurs des revendications 1 à 11, caractérisé par le fait que la substance à transporter adhère au microprojectile par interactions d'adsorption ou par liaisons covalentes ou ioniques de telle sorte que la substance à transporter ne soit pas ou pas complètement désorbée au moment de la traversée de tissu conjonctif, du liquide entourant les cellules ou des couches de cellules, mais continue d'adhérer au microprojectile, jusqu'à ce que la substance à transporter s'immobilise avec le microprojectile dans la cellule-cible.

13. Microprojectile selon la revendication n° 12, caractérisé par le fait qu'il se compose d'un des matériaux or, or de finition microcristallin, céramique oxydée, vitrocéramique ou verre et que l'acide nucléique à transporter est lié au microprojectile de manière covalente par une fonction thiol ou bisulfure ou bien par liaisons éther, acétal, kétal, aldimine, amide ou ester.

14. Microprojectile selon la revendication n° 12 ou 13, caractérisé par le fait que ledit microprojectile se compose d'un matériau électriquement conductible et que l'acide nucléique est lié au microprojectile de manière covalente par réaction électrochimique d'une fonction thiol ou bisulfure liée par liaison covalente à l'acide nucléique, le microprojectile étant utilisé comme électrode.

15. Microprojectile selon les revendications 12 à 14, caractérisé par le fait qu'il présente une granulation de 0,3 à 3,0 µm.

16. Utilisation dans la thérapie génique ex vivo des constructions d'acide désoxyribonucléique selon une ou plusieurs des revendications 1 à 11.

17. Utilisation dans la thérapie génique ex vivo des microprojectiles selon une ou plusieurs des revendications 12 à 15.

18. Utilisation des constructions d'acide désoxyribonucléique selon une ou plusieurs des revendications 1 à 6 pour la fabrication d'un vaccin destiné au traitement des maladies infectieuses de l'homme et de l'animal.
